# EUROPEAN PATENT APPLICATION

(11) **EP 4 534 077 A1**
(43) Date of publication of application: **09.04.2025**
(21) Application number: 24204605.0
(22) Date of filing: 04.10.2024
(51) Int. Cl.: A61K 9/20, A61K 31/197, A61K 31/4415, A61K 31/51, A61K 31/635, A61K 31/714, A61P 29/00

(54) **COMPOSITION COMPRISING CYANOCOBALAMIN, PYRIDOXINE, THIAMINE, PREGABALIN AND CELECOXIB**

(30) Priority: 05.10.2023 MX 2023011799
(71) Applicant: Laboratorios Silanes, S.A. de C.V., Mexico, 11000 (MX)
(72) Inventor: González Canudas, Jorge Alejandro, 11000 Ciudad de México (MX); Espinosa Olivares, Marco Antonio, 11000 Ciudad de México (MX); Muñoz Martínez, Cecilia Jannette, 11000 Ciudad de México (MX); Ollervides Rubio, Paola Yazmín, 11000 Ciudad de México (MX); Sander Padilla, Guillermo, 11000 Ciudad de México (MX)
(74) Representative: Hoffmann Eitle

(57) **Abstract**

The present invention relates to a simple optimized method that allows the combined manufacture of five active substances: cyanocobalamin, pyridoxine, thiamine, pregabalin and celecoxib in a single solid, stable, immediate release pharmaceutical form for pain. The combined use of drugs that act at distinct levels is associated with greater pain relief and, as they can be used at lower doses, with a reduced risk of adverse effects. In this context, the combined use of B vitamins plus pregabalin and celecoxib would be associated with greater analgesic activity through complementary action mechanisms.

## Description

### FIELD OF THE INVENTION

The present invention relates to pharmaceutical chemistry for the manufacture of medicaments, in particular, for those focused on the control of chronic pain with a neuropathic and nociceptive component.

### BACKGROUND OF THE INVENTION

To date, there is no technology that provides alternative synergistic compositions of a neuropathic predominant analgesic drug such as pregabalin, a nociceptive predominant analgesic drug such as celecoxib and their combination with B vitamins (in particular, vitamin B12 and/or vitamin B1 and/or vitamin B6) in the same dosage unit for the treatment of pain.

The document REYES-GARCíA G. ET AL., "Oral Administration of B Vitamins Increases the Antiallodynic Effect of Gabapentin in the Rat;" Proc. West Pharmacol. Soc. 47; published in 2004 describes a study evaluating in a neuropathic pain model the synergistic interaction between gabapentin and a vitamin component comprising vitamins B1, B6, B12 or mixtures thereof. The results of the study show that oral administration of B vitamins induces a modest dose-dependent reduction of tactile allodynia, while coadministration of gabapentin/vitamin B produces a significant dose-dependent reduction of tactile allodynia, where the effective dose 50 ED₅₀ of the gabapentin/vitamin B combination is lower than the additive theoretical dose.

The international patent application publication WO 2009/046801, dated April 16, 2009, refers to a pharmaceutical composition for the treatment of neuropathic pain comprising the vitamin B1 derivative "benfotiamine" and an analgesic (pregabalin); it is indicated that the synergistic interaction of said combination is significantly greater than those recorded for combinations of gabapentin, diclofenac, dexamethasone or ketorolac with B vitamins.

The document MIXCOATL-ZECUATL T. ET AL., "Synergistic Antiallodynic Interaction between Gabapentin or Carbamazepine and either Benfotiamine or Cyanocobalamin in Neuropathic Rats;" Methods Find Exp Clin. Pharmacol. 30 (6), published in 2008, reports the synergistic interaction in compositions comprising gabapentin or carbamazepine and vitamin B12 "cyanocobalamin" or the B1 derivative "benfotiamine" for the treatment of neuropathic pain.

The document REYES-GARCíA G. ET AL., "Analgesic Effect of B Vitamins in Formalin-Induced Inflammatory Pain;" Proc. West. Pharmacol. Soc. 44, published in 2001 describes a study evaluating the analgesic effect of vitamins B1, B6 and B12 or a combination of these for the treatment of pain of inflammatory origin.

The document Romanò CL, Romanò D, Bonora C, Mineo G. "Pregabalin, celecoxib, and their combination for treatment of chronic low-back pain" J Orthop Traumatol. 2009 Dec;10(4):185-91 describes the efficacy of the combination of celecoxib and pregabalin for the treatment of chronic low back pain. It concludes that the combination of celecoxib and pregabalin is more effective than monotherapy for chronic low back pain, with similar adverse effects.

The document MX/a/2011/013989 describes an invention that relates to the pharmaceutical combination for the treatment of neuropathic pain, characterized in that it comprises pregabalin (150 mg), (cyanocobalamin (250 mg, vitamin B12); thiamine hydrochloride (100 mg, vitamin B1) and pyridoxine hydrochloride (50 mg, vitamin B6). The incidence of peripheral neuropathy is not known with certainty. However, it has been estimated that its prevalence worldwide is in a range of 25% to 8% of the population and that it affects both sexes at all ages, but the symptoms are unique to everyone in terms of frequency, quality, and intensity of pain. In Mexico, the Ministry of Health indicates that there are around seven million people affected by neuropathic pain and worldwide statistics indicate that approximately 7% of the population suffers from neuropathic pain.

Mexican patent document MX 292254 discloses the administration to a mammal of a combination of compounds, with at least one ligand directed against the calcium channel subunit α2δ and a nonsteroidal anti-inflammatory drug (NSAID), which provide a striking and potent inhibition of the micturition reflex, superior to that obtained by treatment with a ligand α2δ or NSAIDs alone. Ligands α2δ and NSAIDs combinations are useful for treating lower urinary tract disorders and related symptoms. The ligands α2δ preferred are gabapentin and pregabalin. Preferred NSAIDs are celecoxib, diclofenac, diflunisal, flurbiprofen, naproxen, nimesulide or sulindac.

Mexican patent application document PA/a/2004/008175 refers to a combination comprising a selective COX-2 inhibitor or a pharmaceutically acceptable salt thereof and a ligand α2δ, or a pharmaceutically acceptable salt thereof, and valdecoxib; examples of selective COX-2 inhibitors include valdecoxib, refocoxib, and celecoxib; examples of ligands α2δ include gabapentin, pregabalin, (3S,4S)-(1-aminomethyl-3,4-dimethyl-cyclopentyl)acetic acid, and 3-(1-aminomethyl-cyclohexylmethyl)-4H-[1 ,2,4]oxadiazol-5-one hydrochloride; the combinations are useful for treating certain conditions including joint damage, inflammation, pain, and certain diseases such as arthritis.

Mexican patent document MX 336979 refers to pharmaceutical combinations of two antiepileptics, as well as combinations of an antiepileptic and B vitamins, wherein the antiepileptics are selected from pregabalin and/or oxcarbazepine, and the vitamins are selected from vitamin B1 and vitamin B12. It also relates to pharmaceutical compositions containing such combinations and to the use of such compositions for the treatment of neuropathic pain (NP).

International patent application publication WO 2021/111149 refers to a process for preparing a composition in the form of particles of a specified weight, number and volume (10 nm and 700 pm) comprising solid cores and sequential discrete layers, wherein the process comprises the steps of applying an initial layer of coating material to said solid cores by means of a gas deposition technique and discharging the coated particles from the gas deposition reactor and then agitating the coated particles to disaggregate the particles and finally reapplying an additional layer of coating material to the disaggregated particles. The cores comprise a biological active agent that can be a ligand α2δ (pregabalin), an NSAID and a vitamin, for example, or a mixture of any of them. This document refers to the possibility of manufacturing a composition comprising a ligand α2δ (pregabalin), a nonsteroidal anti-inflammatory drug, and a vitamin, but it does not teach or describe that the NSAID can be an active ingredient such as celecoxib, nor the type(s) ofvitamin(s) suitable for the combination.

In addition, based on a search for various active ingredients, separately or in combination, such as cyanocobalamin/pyridoxine/thiamine, the patent MX 293045 is found, which protects the combination of meloxicam/cyanocobalamin/pyridoxine/thiamine. While searching for patents relating to pregabalin and its combination with other active substances, the MX 360289 patent was also found, which protects the use of the combination of pregabalin with tapentadol, which is characterized by having a slow-release active ingredient (tapentadol) and at least one pharmaceutically acceptable carrier, and a second active agent, wherein the second active agent is pregabalin, wherein the pregabalin is present in a prolonged (controlled) release form or in a delayed release form.

Regarding celecoxib, Mexican patent 366118 of More Pharma Corporation S. de RL de CV, claims a synergistic pharmaceutical composition, for oral administration for the treatment of inflammatory pain comprising 357 mg of paracetamol and 50 to 75 mg of celecoxib.

Within the technical patent information, there are no patent documents related to the combination of cyanocobalamin / pyridoxine / thiamine / pregabalin / celecoxib for oral administration, so it is certain that the combination of these 5 active substances described above has a high degree of technological innovation for the treatment and control of chronic neuropathic and nociceptive pain.

Thus, until now, a stable, immediate-release, fixed-dose combination had not been formulated for the manufacture of a medicine comprising the drugs cyanocobalamin, pyridoxine, thiamine, pregabalin and celecoxib useful in the relief of pain with a chronic neuropathic and nociceptive component, for example, an acute and recurrent painful neuropathy.

### SUMMARY OF THE INVENTION

The present invention relates to a fixed-dose combination pharmaceutical composition of cyanocobalamin, pyridoxine, thiamine, pregabalin and celecoxib with doses of 0.5 mg / 50 mg / 100 mg / 150 mg / 200 mg respectively, and pharmaceutically acceptable carriers in a single solid, stable, immediate-release pharmaceutical form.

Pharmaceutically acceptable carriers are selected from the group comprising surfactants and/or humectants, diluents, solubilizers, binders, disintegrants, dyes, alkalinizing, lubricants and solvents.

In one embodiment, the preferred pharmaceutical form is a tablet.

The present invention also relates to a simple optimized method that allows the combined manufacture of a fixed dose of the five active substances: cyanocobalamin, pyridoxine, thiamine, pregabalin and celecoxib and pharmaceutically acceptable carriers, in a single solid, stable, immediate-release pharmaceutical form for the treatment of pain.

In another embodiment of the invention, the use of the active ingredient cyanocobalamin, pyridoxine, thiamine, pregabalin and celecoxib is described for the manufacture of a medicament useful in the treatment and control of chronic pain with a neuropathic and nociceptive component, for example, an acute and recurrent painful neuropathy, wherein the medicament is a single solid, stable, immediate-release pharmaceutical form. The combined use of drugs that act at distinct levels is associated with greater pain relief and, as they can be used at lower doses, with a reduced risk of adverse effects. In this context, the combined use of B vitamins plus pregabalin and celecoxib would be associated with greater analgesic activity through complementary action mechanisms.

### BRIEF DESCRIPTION OF THE FIGURES

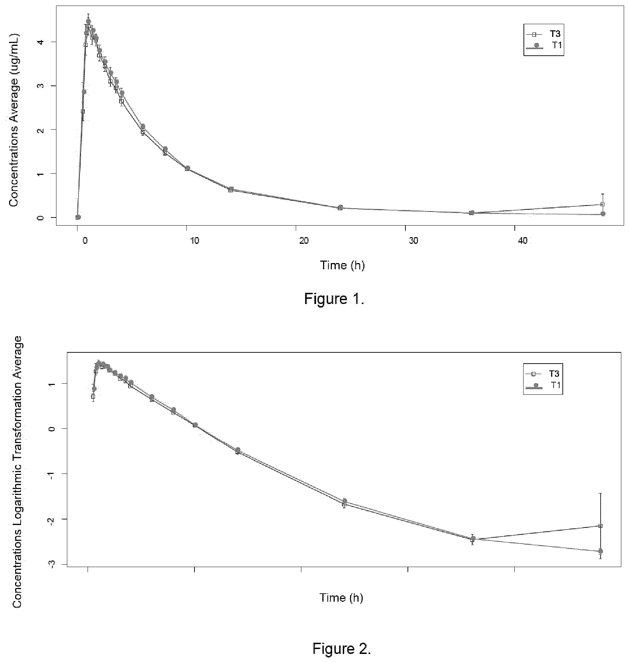

**Figure 1****.** Mean plasma concentrations and standard error in pregabalin. T1: Test medicament celecoxib/pregabalin/vitamin B. T3: Reference medicament, pregabalin.
**Figure 2****.** Log-transformed mean plasma concentrations and standard error of pregabalin. Original concentrations in µg/mL. T1: Test medicament celecoxib/pregabalin/vitamin B. T3: Reference medicament, pregabalin.

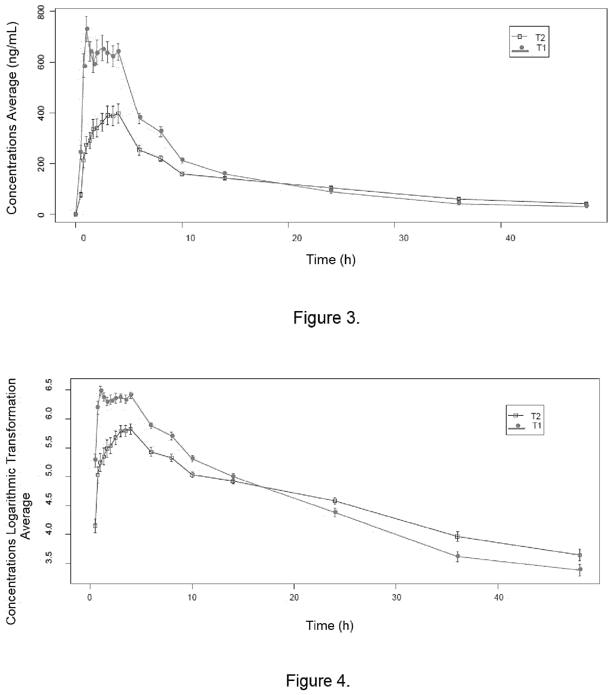

**Figure 3****.** Mean plasma concentrations and standard error for celecoxib. T1: Test medicament celecoxib/pregabalin/vitamin B. T2: Reference medicament, celecoxib.
**Figure 4****.** Log-transformed mean plasma concentrations and standard error of celecoxib. Original concentrations in µg/mL. T1: Test medicament celecoxib/pregabalin/vitamin B. T2: Reference medicament, celecoxib.

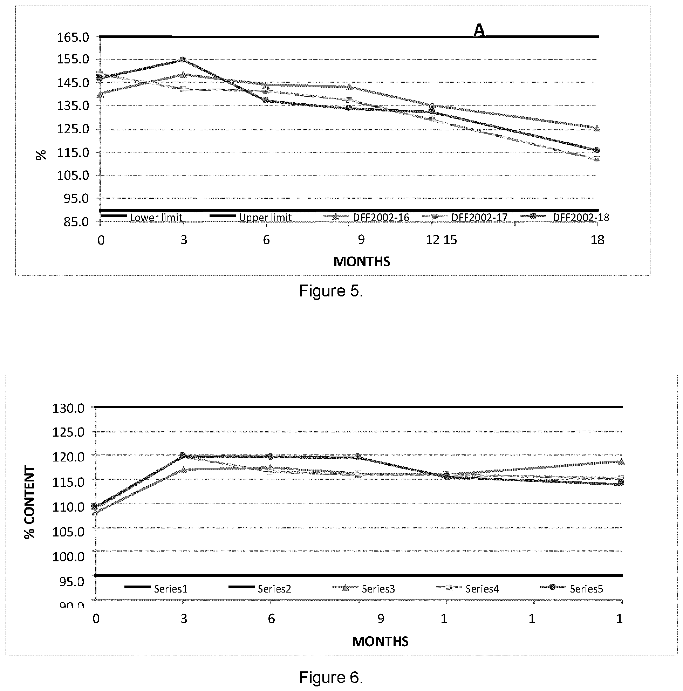

**Figure 5****.** Tendency of cyanocobalamin content results of the product cyanocobalamin - pyridoxine hydrochloride - thiamine mononitrate - pregabalin - celecoxib 0.5 mg / 50 mg / 100 mg / 150 mg / 200 mg tablets, batches DFF2002-16, DFF2002-17 and DFF2002-18, for 18 months under the condition of 30 °C ± 2 °C / 75% RH ± 5% RH.
**Figure 6****.** Tendency of pyridoxine hydrochloride content results of the product cyanocobalamin - pyridoxine hydrochloride - thiamine mononitrate - pregabalin - celecoxib 0.5 mg / 50 mg / 100 mg / 150 mg / 200 mg tablets, batches DFF2002-16, DFF2002-17 and DFF2002-18, for 18 months under the condition of 30 °C ± 2 °C / 75% RH ± 5% RH.

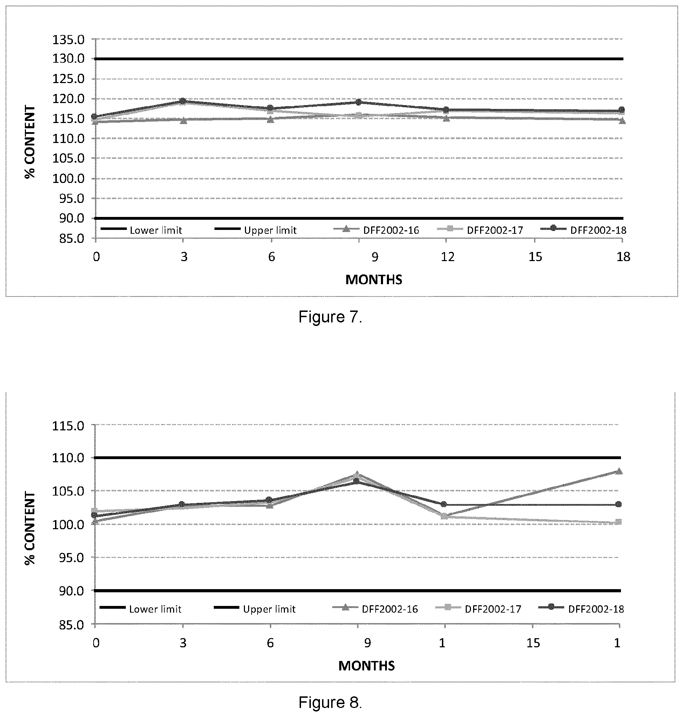

**Figure 7****.** Tendency of thiamine mononitrate content results of cyanocobalamin - pyridoxine hydrochloride - thiamine mononitrate - pregabalin - celecoxib 0.5 mg / 50 mg / 100 mg / 150 mg / 200 mg tablets, batches DFF2002-16, DFF2002-17 and DFF2002-18, for 18 months under the condition of 30 °C ± 2 °C / 75% RH ± 5% RH.
**Figure** 8. Tendency of pregabalin content results of the product cyanocobalamin - pyridoxine hydrochloride - thiamine mononitrate - pregabalin - celecoxib 0.5 mg / 50 mg / 100 mg / 150 mg / 200 mg tablets, batches DFF2002-16, DFF2002-17 and DFF2002-18, for 18 months under the condition of 30 °C ± 2 °C / 75% RH ± 5% RH.

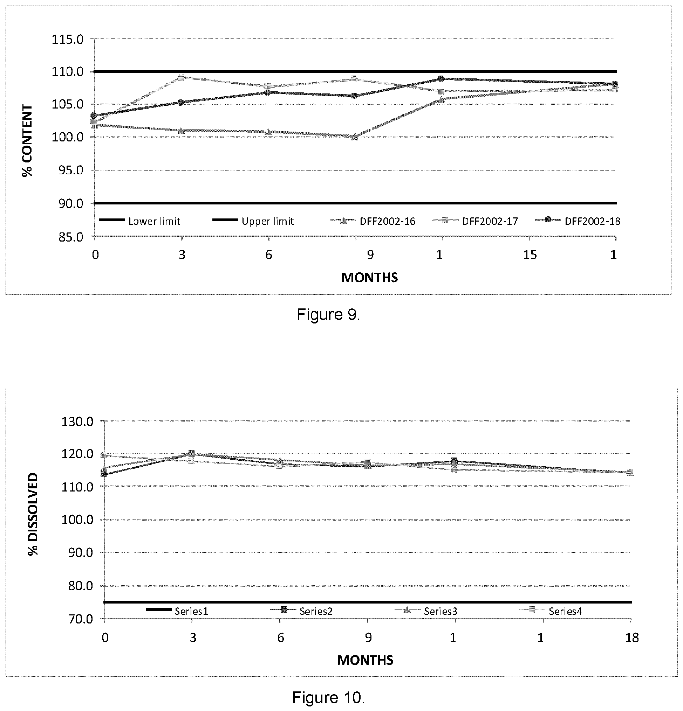

**Figure 9****.** Tendency of celecoxib content results of the product cyanocobalamin - pyridoxine hydrochloride - thiamine mononitrate - pregabalin - celecoxib 0.5 mg / 50 mg / 100 mg / 150 mg / 200 mg tablets, batches DFF2002-16, DFF2002-17 and DFF2002-18, for 18 months under the condition of 30 °C ± 2 °C / 75% RH ± 5% RH.
**Figure 10****.** Tendency of pyridoxine hydrochloride dissolution results of the product cyanocobalamin - pyridoxine hydrochloride - thiamine mononitrate - pregabalin - celecoxib 0.5 mg / 50 mg / 100 mg / 150 mg / 200 mg tablets, batches DFF2002-16, DFF2002-17 and DFF2002-18, for 18 months under the condition of 30 °C ± 2 °C / 75% HR ± 5% HR.

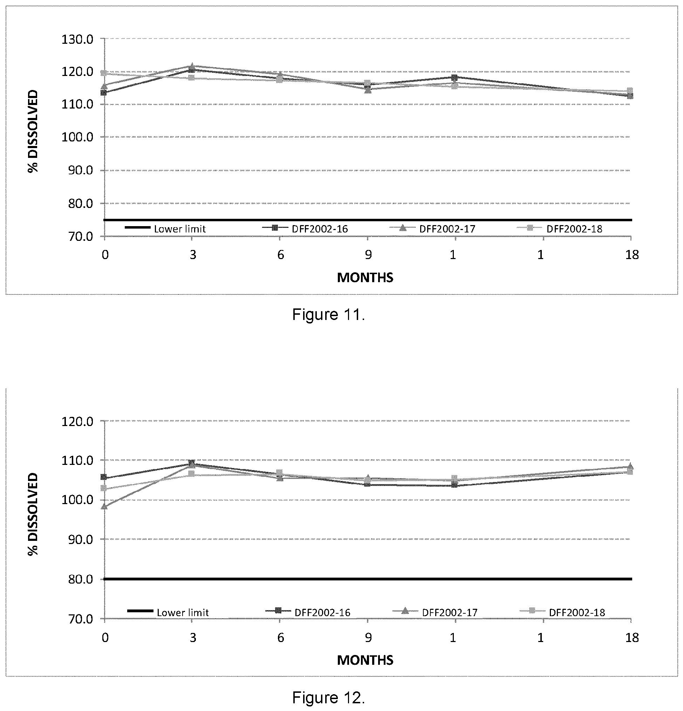

**Figure 11****.** Tendency of thiamine mononitrate dissolution results of cyanocobalamin - pyridoxine hydrochloride - thiamine mononitrate - pregabalin - celecoxib 0.5 mg / 50 mg / 100 mg / 150 mg / 200 mg tablets, batches DFF2002-16, DFF2002-17 and DFF2002-18, for 18 months under the condition of 30 °C ± 2 °C / 75% RH ± 5% RH.
**Figure 12****.** Tendency of pregabalin dissolution results of the product cyanocobalamin - pyridoxine hydrochloride - thiamine mononitrate - pregabalin - celecoxib 0.5 mg / 50 mg / 100 mg / 150 mg / 200 mg tablets, batches DFF2002-16, DFF2002-17 and DFF2002-18, for 18 months under the condition of 30 °C ± 2 °C / 75% RH ± 5% RH.

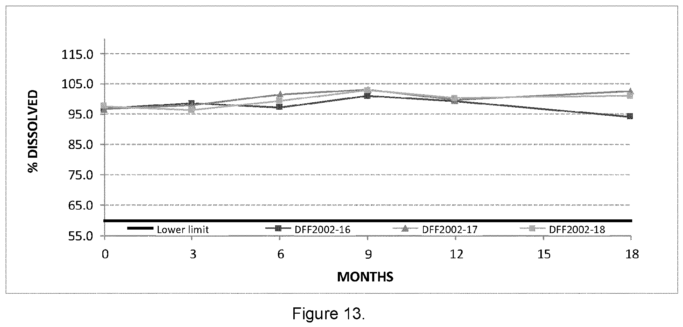

**Figure 13****.** Tendency of celecoxib dissolution results of the product cyanocobalamin - pyridoxine hydrochloride - thiamine mononitrate - pregabalin - celecoxib 0.5 mg / 50 mg / 100 mg / 150 mg / 200 mg tablets, batches DFF2002-16, DFF2002-17 and DFF2002-18, for 18 months under the condition of 30 °C ± 2 °C / 75% RH ± 5% RH.

### DETAILED DESCRIPTION OF THE INVENTION

The manufacture of a pharmaceutical composition for oral administration comprising the combination of cyanocobalamin, pyridoxine, thiamine, pregabalin and celecoxib is described. The method allows obtaining a pharmaceutical composition that is easy to administer in a single dose. The present invention relates both to the pharmaceutical composition and to the manufacturing method for obtaining it.

The technological development involved a series of evaluations defining the formulation and the process as described below. The product combines in a single dosage unit the drugs cyanocobalamin, pyridoxine, thiamine, pregabalin and celecoxib, with doses of 0.5 mg / 50 mg / 100 mg / 150 mg / 200 mg, respectively, and pharmaceutically acceptable vehicles (see Table 1).

The technological complexity lies in the combination of the five drugs, with different rheological and solubility characteristics, together with the pharmaceutically acceptable vehicles.

On the one hand, there is a liposoluble drug, with poor flow and excessive adhesiveness (celecoxib) and on the other, there is hydro soluble drugs, with poor flow and low compaction (pyridoxine, thiamine), which could cause interference in stability, absorption, and release mechanism.

### Cyanocobalamin

Condensed formula: C₆₃H₈₈CoN₁₄O₁₄P
Description: Dark red crystals or red amorphous powder. The anhydrous form is hygroscopic.
pH: 6.0 (1 g/L at 20 °C)
pKa: 1.82
Solubility: Soluble in alcohol, slightly soluble in water; almost insoluble in acetone, chloroform, and diethyl ether. 12 g/L

### Pyridoxine

Condensed formula: C₈H₁₁NO₃O · HCl
Description: White or almost white crystalline powder, stable in air and slowly decomposes with light.
pKa: 9.4
Solubility: Easily soluble in water; slightly soluble in alcohol; almost insoluble in diethyl ether.

### Thiamine

Condensed formula: C₁₂H₁₇N₅O₄S
Description: White or almost white crystalline powder or small colorless crystals.
pKa: 15.5
Solubility: Freely soluble in hot water, slightly soluble in water, slightly soluble in ethanol and methanol, very slightly soluble in chloroform, almost insoluble in diethyl ether.

### Pregabalin

Condensed formula: C₈H₁₇NO₂
Description: White crystals without characteristic odor or taste.
Solubility: 11.3 g/L.
Biopharmaceutical classification: Classification I, high permeability, and high solubility.

### Celecoxib

Condensed formula: C₁₇H₁₄F₃N₃O₂S
Description: White crystals without characteristic odor or taste.
Solubility: 3.3 g/L.
Biopharmaceutical classification: Classification II, high permeability, and low solubility.

On the other hand, a series of tests were conducted in which the components of the formulation were selected based on their function and concentration. It was established in relation to the product's behavior, ensuring the proper functioning of the product.

### Surfactant / humectant

It is selected from the group comprising poloxamers, polyoxyethylene castor oil derivatives, benzalkonium chloride, benzethonium chloride, polyoxyethylene alkyl ethers and polyoxyethylene sorbitan fatty acid esters, preferably the surfactant or humectant is sodium lauryl sulfate, which is added in the range of 1 to 25%. Sodium lauryl sulfate can be added at a concentration of 0.64% which allows the insoluble drug to be moistened, breaking the surface tension and achieving a solubilizing effect.

### Binder

It is selected from the group comprising povidones in all K grades and their derivatives, cellulose derivatives such as carbomers, hydroxypropyl celluloses, carboxymethyl celluloses, starch derivatives such as pregelatinized starch and corn starch, and is added in the range of 1 to 25%. The binder can also be a polyvidone, preferably a polyvidone K30 that can be added in a concentration of 3.27%, a high binding power additive, which by absorbing water during manufacturing promotes the granulation of the components of the formulation by forming bonds between the particles of the materials, improving the physical properties of the powder mixture, such as compaction, particle size, density and flow.

### Diluent

It is selected from the group comprising cellulose derivatives such as microcrystalline cellulose, phosphate derivatives such as dibasic calcium phosphates, starch derivatives such as pregelatinized starch and corn starch and even lactose such as anhydrous lactose and is added in a range of 5 to 90%. The diluent can thus be a microcrystalline cellulose, preferably a silicified microcrystalline cellulose, which can be added at a concentration of 27.99% and which, due to its improved compaction properties in direct compression compared to conventional microcrystalline cellulose, provides stability to each of the extra granular drugs. The diluent can also be a lactose, preferably a lactose monohydrate that can be added in a concentration of 16.33% and that due to its good flow characteristics and particle size is useful for the insoluble drug (Celecoxib), since it makes the mixture more hydrophilic, and it does not present incompatibility problems with the other components of the formulation.

### Disintegrant

It is selected from the group comprising cellulose derivatives such as, for example, hydroxypropyl cellulose, carboxymethyl cellulose, microcrystalline cellulose; povidone derivatives such as crospovidone, plastons; starch derivatives for example pregelatinized starch, sodium starch glycolate and corn starch, and is added in a range of 0.5 to 15%. The disintegrant may also be croscarmellose sodium, a white to greyish odorless powder, which can be added at a concentration of 4.08% to increase the disintegration rate of the tablet and therefore improve the dissolution profile of the drugs.

### Lubricant

It is selected from the group comprising stearate derivatives such as, for example, magnesium stearate, zinc stearate, calcium stearate, stearic acid, monostearates, stearyl fumarate; sulfated derivatives such as, for example, magnesium lauryl sulfate and magnesium lauryl sulfate, talc, and are added in a range of 0.25 to 10%. The lubricant is preferably magnesium stearate which can be added in a concentration of 0.41% to reduce friction and prevent sticking between the different tools during the compression process.

### Dye

It is selected from the group comprising beta-carotene; indigo carmine, iron oxides, sunset yellow FCF and titanium dioxide, and is added in a range of 0.5 to 1.0% (DL50 (rat, oral): 10 g/kg). The dye is preferably yellow No. 5, which can be added at a concentration of 0.31% and, due to its stable conditions, provides a pleasant color in combination with cyanocobalamin.

### Solubilizer

It is selected from the group comprising different grades of polyethylene glycol, glyceryl behenate, microcrystalline wax and stearoyl polyoxyglycerides, and is added in a range of 5 to 10%. The solubilizer can also be a poloxamer, preferably a poloxamer 188 that can be added in a concentration of 2.04% which emulsifying properties give it a solubilizing function.

### Alkalizing

It is selected from the group comprising sodium carbonate, calcium hydroxide, potassium bicarbonate, potassium citrate, potassium hydroxide, sodium citrate dihydrate and sodium hydroxide, and is added in a range of 10 to 50%. The alkalizing agent can also be a bicarbonate, preferably sodium bicarbonate, which can be added in a concentration of 4.08% to increase the bioavailability of the drug in the body.

The selection of the additives was conducted considering the physicochemical characteristics of the drugs. For celecoxib, its rheological and adhesive characteristics are improved, as well as its intrinsic stability by modulating the pH, generating a microenvironment that facilitates its dissolution and therefore its bioavailability. For pregabalin and vitamins, their rheological and compaction characteristics are improved, ensuring their stability and dissolution.

Once the drugs or active substances cyanocobalamin, pyridoxine, thiamine, pregabalin and celecoxib have been selected, with fixed doses of 0.5 mg / 50 mg / 100 mg / 150 mg / 200 mg, respectively, and pharmaceutically acceptable vehicles for a single dosage unit were defined.

The pharmaceutical form selected for dosing the drugs was "tablets" due to their dosage accuracy and because it is the most widely accepted pharmaceutical form due to its easy administration. Capsules are an alternative; however, it is not possible to dose such a high concentration of drugs due to the limited size of the capsules. In the case of tablets, it is possible to reduce the volume of the powders and thus facilitate their handling and administration.

The manufacturing process of the pharmaceutical composition comprises the following steps:
i. mixing for 10 minutes the active ingredient celecoxib with a first diluent, a disintegrant, a binder, a solubilizer and an alkalinizing previously sifted;
ii. moistening with purified water and granulating;
iii. drying the granule obtained in the previous step until reaching a humidity less than or equal to approximately 0.8%;
iv. adding and mixing for 5 minutes the active ingredient cyanocobalamin and a portion of a second diluent;
v. adding and mixing for 5 minutes a portion of a second diluent;
vi. repeating the previous step by adding and mixing for 5 minutes a portion of a second diluent;
vii. adding and mixing for 5 minutes a solubilizer;
viii. adding the active ingredients pyridoxine, thiamine and pregabalin, and a portion of a second diluent;
ix. adding and mixing for 5 minutes a portion of a second diluent;
x. adding and mixing for 5 minutes a binder, a surfactant/humectant and a disintegrant;
xi. adding and mixing for 3 minutes a previously sifted dye and lubricant; and
xii. compressing the granulate obtained in the previous step.

In a preferred embodiment the process comprises:
- in step i., 50% of the total amount of the disintegrant, 50% of the total amount of the binder, and 50% of the total amount of the solubilizer are mixed;
- in step iv., the portion of the second diluent is 5% of the total amount of the second diluent;
- in step v., the portion of the second diluent is 10% of the total amount of the second diluent;
- in step vi., the portion of the second diluent is 20% of the total amount of the second diluent;
- in step vii., the remaining 50% of the solubilizer is mixed;
- in step viii., 32.5% of the total amount of the second diluent is mixed;
- in step ix., the portion of the second diluent is 32.5% of the total amount of the second diluent;
- in step x., The remaining 50% of the binder and the remaining 50% of the disintegrant are mixed.

In a preferred embodiment, the pyridoxine is pyridoxine hydrochloride, while the thymine is thymine mononitrate.

In a non-limiting embodiment, the surfactant/humectant, binder, diluent, disintegrant, lubricant, dye, solubilizer, and alkalinizing are selected from the options detailed above.

In a preferred embodiment, the surfactant/humectant is sodium lauryl sulfate, the first diluent is lactose monohydrate, the solubilizer is poloxamer, the binder is polyvidone, the disintegrant is croscarmellose sodium, the second diluent is microcrystalline cellulose, the dye is yellow No. 5, the alkalinizing is sodium bicarbonate, and the lubricant is magnesium stearate.

The method of the present invention, which allows obtaining a single product that combines the dosage of the drugs cyanocobalamin, pyridoxine, thiamine, pregabalin and celecoxib, represents a set of important technological challenges due to the physicochemical properties of each active ingredient and the difference in dosage, to guarantee obtaining a stable product.

In another aspect of the present invention, a method is provided for the treatment and control of chronic pain, which comprises administering a composition according to the present invention to a patient in need thereof. In a preferred embodiment, the pain is a painful, acute and recurrent neuropathy.

### EXAMPLES

### Example 1. Preparation of a pharmaceutical composition according to an embodiment of the present invention.

A pharmaceutical composition was prepared with the qualitative-quantitative formula described in Table 1.

**Table 1. Qualitative-quantitative formula of a multi-active tablet in the treatment and control of chronic pain**

| **mg/Tab** | **Components** | **Function** |
|---|---|---|
| 0.50 | Cyanocobalamin | Drug |
| 50.00 | Pyridoxine HCl | |
| 100.00 | Thiamine mononitrate | Drug |
| 150.00 | Pregabalin | Drug |
| 200.0 | Celecoxib | Drug |
| 7.80 | Sodium lauryl sulfate | Surfactant / humectant |
| 200.00 | Lactose monohydrate | Diluent |
| 25.00 | Poloxamer 188 | Solubilizer |
| 40.00 | Polyvidone K30 | Binding agent |
| 50.00 | Croscarmellose sodium | Disintegrant |
| 342.90 | Silicified microcrystalline cellulose | Diluent |
| 3.80 | Yellow color 5 | Dye |
| 50.00 | Sodium bicarbonate | Alkalizing agent |
| 500 | Magnesium stearate | Lubricant |
| 0.068 | Purified water (mL) | Solvent |

For the manufacture of the pharmaceutical composition described in Table 1, a process comprising the following steps was followed:
i. A sifting operation was performed through a 0.157 cm mesh (0.062 inches) on the following materials: celecoxib (active ingredient), lactose monohydrate (diluent), 50.0% croscarmellose sodium (disintegrant), 50.0% polyvidone K30 (binder), 50% poloxamer 188 (solubilizer) and sodium bicarbonate (alkalinizing).
ii. The sieve from step No. 1 was added to a mixer and mixed for 10 minutes.
iii. The mixture from step No. 2 was added to a granulator; it is moistened and granulated with purified water.
iv. In the granulating equipment, the granule obtained from step No. 3 was dried until reaching a humidity less than or equal to 0.8%.
v. A size reduction operation was performed by 0.127 cm mesh (0.050 inches) on the granules obtained in step No. 4.
vi. Cyanocobalamin (active ingredient) and 5.0% silicified microcrystalline cellulose (diluent) were added to a diffusion mixer. It was mixed for 5 minutes.
vii. 10% silicified microcrystalline cellulose (diluent) was added to the diffusion mixer of step No. 6. Mix for 5 minutes.
viii. 20% silicified microcrystalline cellulose (diluent) was added to the diffusion mixer of step No. 7. Mix for 5 minutes.
ix. 50% poloxamer 188 (solubilizer) was added to the diffusion mixer of step No. 8. It was mixed for 5 minutes.
x. To the diffusion mixer of step No. 9, the following materials were added: pyridoxine hydrochloride (active ingredient), thiamine mononitrate (active ingredient), pregabalin (active ingredient) and 32.5% silicified microcrystalline cellulose (diluent). It was mixed for 5 minutes.
xi. To the diffusion mixer of step No. 10 was added 32.5% of silicified microcrystalline cellulose (diluent). It was mixed for 5 minutes.
xii. A sifting operation was performed through a 0.157 cm mesh (0.062 inches) on the following materials: 50.0% polyvinyl chloride K30 (binder), sodium lauryl sulfate (surfactant/humectant) and 50.0% croscarmellose sodium (disintegrant).
xiii. The sieves from steps No. 5 and 12 were added to a diffusion mixer, it was mixed for 5 minutes.
xiv. A sifting operation was performed through a 0.157 mesh (0.062 inches); yellow No. 5 (dye) and magnesium stearate (lubricant) were added.
xv. The mixture from step No. 13 and the sieving from step No. 14 were added to a diffusion mixer, it was mixed for 3 minutes.
xvi. From the granules, tablets with good mechanical characteristics are obtained; thus, the final powder mixture was compressed to 1225 mg ± 61 mg, with a hardness of no less than 14.0 Kp and a disintegration time of no more than 30 minutes.

Thus, manufacturing was conducted considering the physicochemical characteristics of the drugs; for celecoxib, its rheological characteristics and adhesiveness were improved; for the other active ingredients, the rheological and compaction characteristics were improved, ensuring their stability and dissolution.

### Example 2. Comparative bioavailability clinical trial

### Pharmacokinetic non-interaction study between celecoxib 200 mg/pregabalin 150 mg/vitamin B, administered individually or in combination, single dose in healthy subjects, both genders under fasting conditions

In the present study, the pharmacokinetic profile (Cₘₐₓ, AUC₀₋ₜ, AUC_{0-∞}, t^{max}, Kₑ, and t_{1/2}) of the combination celecoxib 200 mg / pregabalin 150 mg / vitamin B single dose (tablet, test drug) manufactured by Laboratorios Silanes S.A. de C.V., versus each component administered individually (celecoxib 200 mg and pregabalin 150 mg, reference drugs), was compared in healthy subjects of both sexes, under fasting conditions to establish the non-pharmacokinetic interaction of the drugs in combination, and to check whether the 90% confidence intervals for the test drug and the reference drugs are within the range of 80-120% for Cₘₐₓ and AUC₀₋ₜ.

The study was open, longitudinal, prospective, with a 3x6x3 design, with random assignment, single dose, three treatments, three periods and six sequences under fasting conditions, in a healthy Mexican female and male population with a washout time for drug elimination of one week (07 days) between each period.

**Table 2. Test medicament and reference medicament.**

| | **Test medicament** | **Reference medicament 1** | **Reference medicament 2** |
|---|---|---|---|
| International nonproprietary name | Celecoxib-Pregabalin-Vitamin B | Celecoxib | Pregabalin |
| Generic name | Celecoxib-Pregabalin-Vitamin B | Celecoxib | Pregabalin |
| Pharmaceutical Form | Tablet | Capsule | Capsule |
| Dose | Celecoxib 200 mg | 200 mg | 150 mg |
| | Pregabalin 150 mg | | |
| | Vitamin B12 500 µg | | |
| | Vitamin B1 100 mg | | |
| | Vitamin B6 50 mg | | |
| Batch | 20F082G1 | X86082 | |
| Manufacturer | Laboratorios Silanes S.A. de C.V. | Pfizer | Pfizer |

The study was conducted in two groups and three periods; the study began with 42 research subjects and ended with 41 female (27) and male (14) subjects who met the inclusion criteria and none of the established exclusion criteria. Due to the COVID-19 health emergency, the 42 research subjects were divided into two hospitalization groups: Group 1 with 22 research subjects and Group 2 with 20 research subjects. After fasting for at least 10 hours, a blood sample was taken at time 0.00 hours, and a dose of celecoxib / pregabalin / vitamin B, 200 mg / 150 mg tablets / vitamin B12 500 µg / vitamin B1 100 mg / vitamin B6 50 mg was administered, in a single dose of the test medicament or the reference medicament as monotherapy (celecoxib 200 mg capsule or pregabalin 150 mg capsule) according to prior randomization, orally with 250 mL of water at room temperature. After administration of the corresponding medicament, another 17 samples were taken at the following times: 0.50, 0.75, 1.00, 1.33, 1.66, 2.00, 2.50, 3.00, 3.50, 4.00, 6.00, 8.00, 10.00, 14.00, 24.00, 36.00- and 48.00-hours post-dose.

The analytical methods for the plasma quantification of celecoxib and pregabalin were developed and validated in accordance with the state of the art well known to experts in the matter and following the parameters of NOM-177-SSA1-2013, as well as for the results obtained during the analysis of the samples, while the storage conditions of the plasma samples were -65 °C ± 15 °C. During the analysis of the samples, the methods proved to be linear, reproducible, precise, and specific.

A total of 25 adverse events probably related to celecoxib/pregabalin/vitamin B dosing occurred (20 events of dizziness, 2 events of visual disturbances, 1 event of nausea, and 2 events of paresthesia) and 16 adverse events probably related to pregabalin dosing occurred (13 events of dizziness, 1 event of headache, 1 event of visual disturbances, and 1 event of paresthesia). All adverse events were classified as non-serious, 44 of 45 events were classified as mild in severity, only 1 event was considered moderate in severity. All adverse events ended without the presence of additional complications or sequels.

The average values of the pharmacokinetic parameters (Tables 3-4), their respective arithmetic means, standard deviations, coefficient of variation, geometric mean, minimum, median, and maximum of the estimated pharmacokinetic parameters from the sampling of the treatments were as follows:

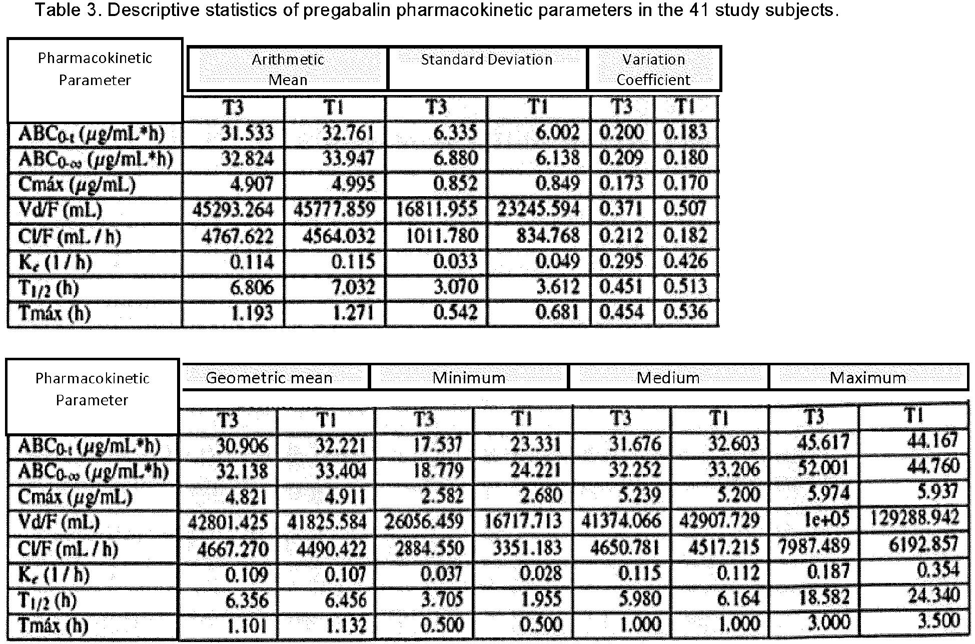

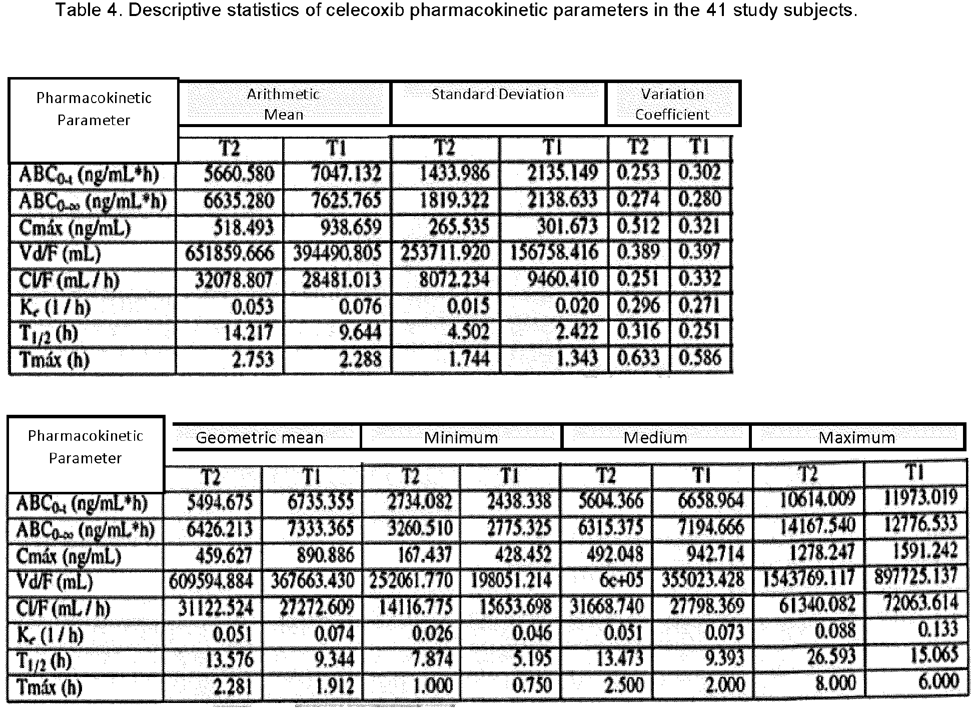

### 90% confidence intervals, in pregabalin with the 41 patients

The 90% confidence intervals for the logarithmic transformation of Cₘₐₓ, ABC₀₋ₜ and ABC_{0-∞} are in the range of 80 to 125% cited in the protocol; therefore, the null hypothesis of no bioavailability can be rejected (Tables 5-6).

**Table 5. 90% confidence intervals of pregabalin pharmacokinetic parameters**

| **Parameters\|**** | **Classic Interval** | | **Power** |
|---|---|---|---|
| ABC₀₋ₜ | 100.8535 | 107.6562 | 0.9999 |
| ABC_{0-∞} | 100.2297 | 107.6082 | 0.9999 |
| Cmax | 96.8889 | 107.0803 | 0.9293 |

| | | | |
|---|---|---|---|
| **Logarithmic transformation base e. | | | |

*90% confidence intervals for celecoxib with the 41 patients*

The 90% confidence intervals for the logarithmic transformation of Cₘₐₓ and ABC₀₋ₜ, are not in the range of 80 a 125%. However, ABC_{0-∞}, if it is found within the range cited in the protocol, the null hypothesis of non-equivalence in bioavailability cannot therefore be rejected.

**Table 6. 90% confidence intervals of celecoxib pharmacokinetic parameters.**

| **Parameter**** | **Classic Interval** | | **Power** |
|---|---|---|---|
| ABC₀₋ₜ | 113.5303 | 124.2737 | 0.9999 |
| ABC_{0-∞} | 105.3952 | 115.7104 | 0.9999 |
| Cmax | 166.0946 | 212.8574 | 0.9073 |

*Graphs of mean plasma concentrations of pregabalin* with the 41 subjects can be seen in Figures 1 and 2; while the graphs of the average plasma concentrations of celecoxib with the 41 subjects can be seen in Figures 3 and 4.

### Statistical conclusion

According to the comparative bioavailability tests used and suggested by the NOM-177-SSA1 -2013 standard in the comparison of the pregabalin substance of the fixed combination celecoxib / pregabalin / vitamin B against individual pregabalin, the IC90 estimated from the logarithmic transformation of the pharmacokinetic parameters Cₘₐₓ, ABC₀₋ₜ, ABC_{0-∞} are in the range of 80 to 125%, range cited in the protocol. Likewise, the P-values of t1 and t2 of the two-tailed t-test for the parameters Cₘₐₓ, ABC₀₋ₜ, ABC_{0-∞} were less than 0.05, allowing the rejection of the null hypothesis of non-equivalence in bioavailability in the substance evaluated. The rate and amount of absorption of pregabalin was not affected when administered in the combination formulation celecoxib-pregabalin-vitamin B 200 mg/150 mg tablets, manufactured by Laboratorios Silanes SA de CV since its bioavailability was equivalent to that of the individual pregabalin 150 mg tablets.

For the celecoxib substance of the fixed combination celecoxib/pregabalin/vitamin B versus celecoxib alone, the 90% confidence intervals (CI90) estimated from the logarithmic transformation of the pharmacokinetic parameters Cₘₐₓ, ABC₀₋ₜ, are not within the range 80 to 125%, range cited in the protocol; the interval for the pharmacokinetic parameter In(AUC_{0-∞}) is within that range. Similarly, the P-values of t1 of the two-sided t test for the pharmacokinetic parameters Cₘₐₓ, ABC₀₋ₜ were less than 0.05. However, the P-values of t2 of the Schuirmann double-tailed t test for the same parameters were greater than 0.05, thus not allowing the rejection of the null hypothesis of non-equivalence in bioavailability.

Therefore, it is concluded that from a statistical point of view, an interaction is observed between the components of the formulation of the fixed dose combination of celecoxib / pregabalin / vitamin B tablets of 200 mg / 150 mg / / vitamin B12 500 µg / vitamin B1 100 mg / vitamin B6 50 mg, manufactured by Laboratorios Silanes, SA de CV, for the drug celecoxib since its bioavailability expressed in Cₘₐₓ and ABC₀₋t is not equivalent to that observed for the individual reference medicament celecoxib 200 mg capsules (Celebrex^{®}) showing in the case of the fixed combination a bioavailability with a higher absorption speed (Cₘₐₓ) and quantity (ABC₀₋ₜ). However, considering that the values of Cₘₐₓ and ABC₀₋ₜ obtained in this study 938.6 ±301.7 ng/mL and 7047.13 ± 2135.1 ng/mL*h are comparable to those reported for the reference medicament in the literature (Table 7).

**Table 7. Pharmacokinetic parameters of celecoxib reported in the literature.**

| Author | Cₘₐₓ (ng/mL) | ABC₀₋ₜ (ng/mL*h) |
|---|---|---|
| Laboratorios Silanes | 602.685 ± 301.48 | 5593.760 ± 2158.606 |
| Sy-Yeuan J | 703.86 ± 329.91 | 7267.48 ± 2077.68 |
| Lilitkarntrakul, P | 686.83 ± 211.35 | 5157.12 ± 1499.46 |
| Paulson, S | 806.0 ± 411.0 | 5994.0 ± 2393.0 |
| Center for Drug Evaluation, 1999 | 600.0 ± 900.00 | - |

The ABC_{0-∞} was initially mentioned as descriptive. However, the results obtained in this study (7144.894 ng/mL*h) are within the evaluation intervals and are equivalent to those observed in the reference medicament.

However, by removing from the statistical analysis the extreme values with studentized residuals with absolute values greater than 2 (NOM-177-SSA1-2013), the IC90 of In(ABC₀₋ₜ) and In(ABC_{0-∞}) remain in the range 80 to 125%, range cited in the protocol.

It is argued that the possible interaction observed in the fixed combination has no clinical relevance for either safety or efficacy, the above also supported by the fact that the drug pregabalin has no effect on the induction or inhibition of liver enzymes, which is the main route of celecoxib metabolism, and which consequently would be the explanation for these results. The observed results could be explained by the difference in pharmaceutical form between the fixed combination (tablet) and the reference medicament (capsule). Regarding the bioavailability of pregabalin in the fixed combination (Cₘₐₓ, ABC₀₋ₜ, ABC_{0-∞}) was observed to be statistically equivalent to that of the reference drug pregabalin capsules 150 mg (Lyrica^{®}) supporting the non-interaction between the medicaments.

Finally, since no serious adverse events occurred, it is concluded that the medicament celecoxib/pregabalin/vitamin B 200 mg/150 mg tablet from Laboratorios Silanes, SA de CV is safe for dosing in humans.

### Example 3. Stability of the pharmaceutical composition

### Long-term stability condition of the product cyanocobalamin - pyridoxine hydrochloride - thiamine mononitrate - pregabalin - celecoxib 0.5 mg / 50 mg / 100 mg / 150 mg / 200 mg tablets

This study was conducted in accordance with the requirements established in the Mexican Official Standard NOM-073-SSA1-2015, Stability of drugs and medicaments, the Mexican Official Standard NOM-059-SSA1-2015, numeral 11, which includes the requirements for good practices in analytical laboratories, as well as the guidelines established in the WHO Expert Committee on Specifications for Pharmaceutical Preparations Forthy Third Report, WHO.

The stability of the product cyanocobalamin - pyridoxine hydrochloride - thiamine mononitrate - pregabalin - celecoxib 0.5 mg / 50 mg / 100 mg / 150 mg / 200 mg tablets, batches DFF2002-16, DFF2002-17, DFF2002-18 (manufactured in Laboratorios Silanes SA de CV), was evaluated under the condition of long-term stability for climatic zone IVB (30 °C ± 2 °C / 75 % ± 5 % RH) for 18 months.

The batch size was:
- DFF2002-16: 25,000 tablets in a cold-formed aluminium blister of 176 µm / aluminium foil 25 µm.
- DFF2002-17: 25,000 tablets in a cold-formed aluminium blister of 176 µm / aluminium foil 25 µm.
- DFF2002-18: 25,000 tablets in a cold-formed aluminium blister of 176 µm / aluminium foil 25 µm.

The parameters analyzed include:
- Description
- Cyanocobalamin content
- Pyridoxine hydrochloride content
- Thiamine mononitrate content
- Pregabalin content
- Celecoxib content
- Pyridoxine hydrochloride solution
- Thiamine mononitrate solution
- Pregabalin solution
- Celecoxib dissolution
- Organic impurities celecoxib
- Related substances pregabalin
- Microbial limits
- Water content
- Hardness

The results are described below in Tables 8 to 10. All batches were shown to retain their chemical and physical characteristics within established specifications, thereby ensuring their quality, efficacy, and safety.

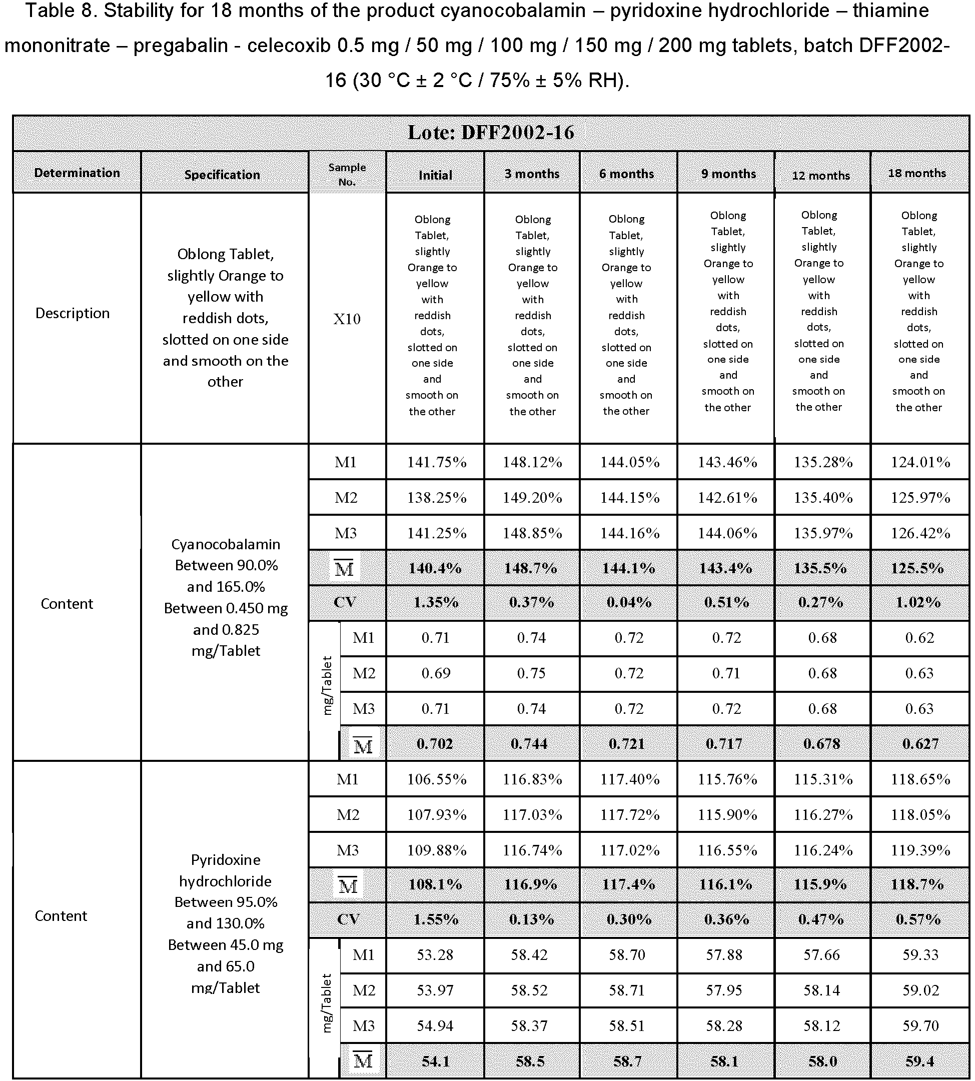

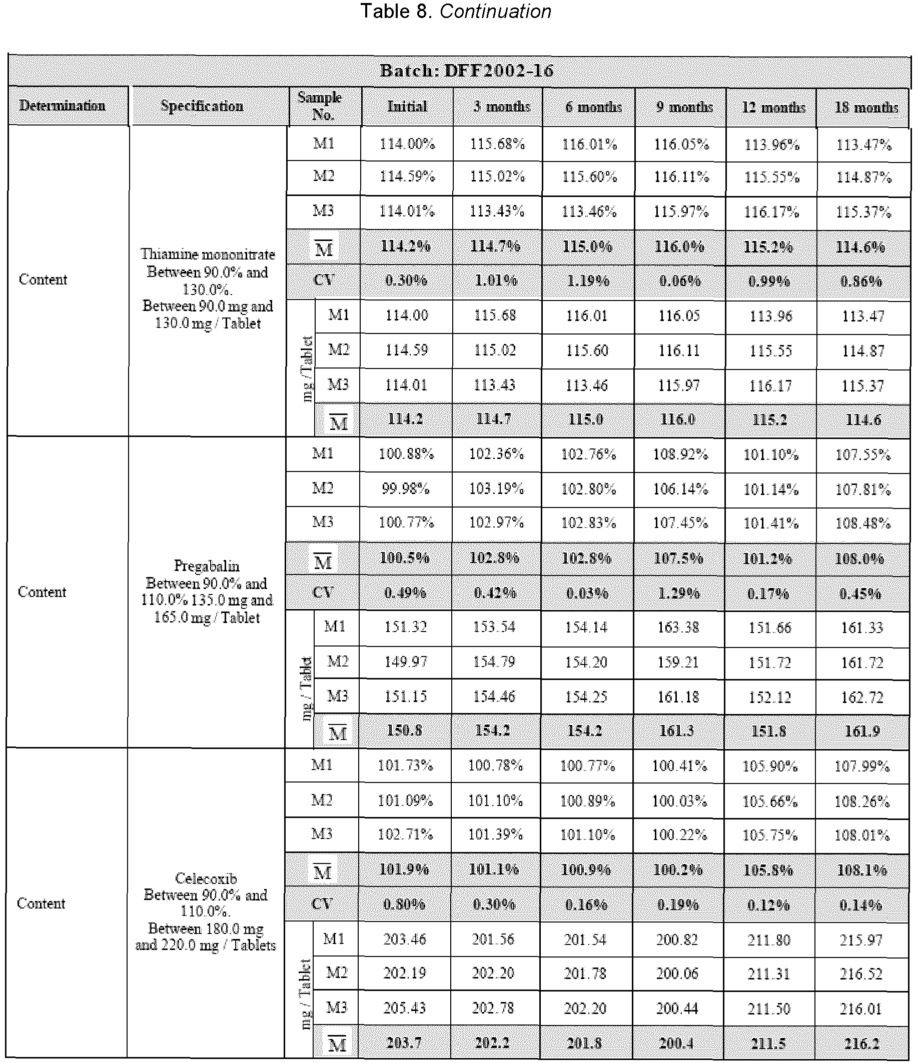

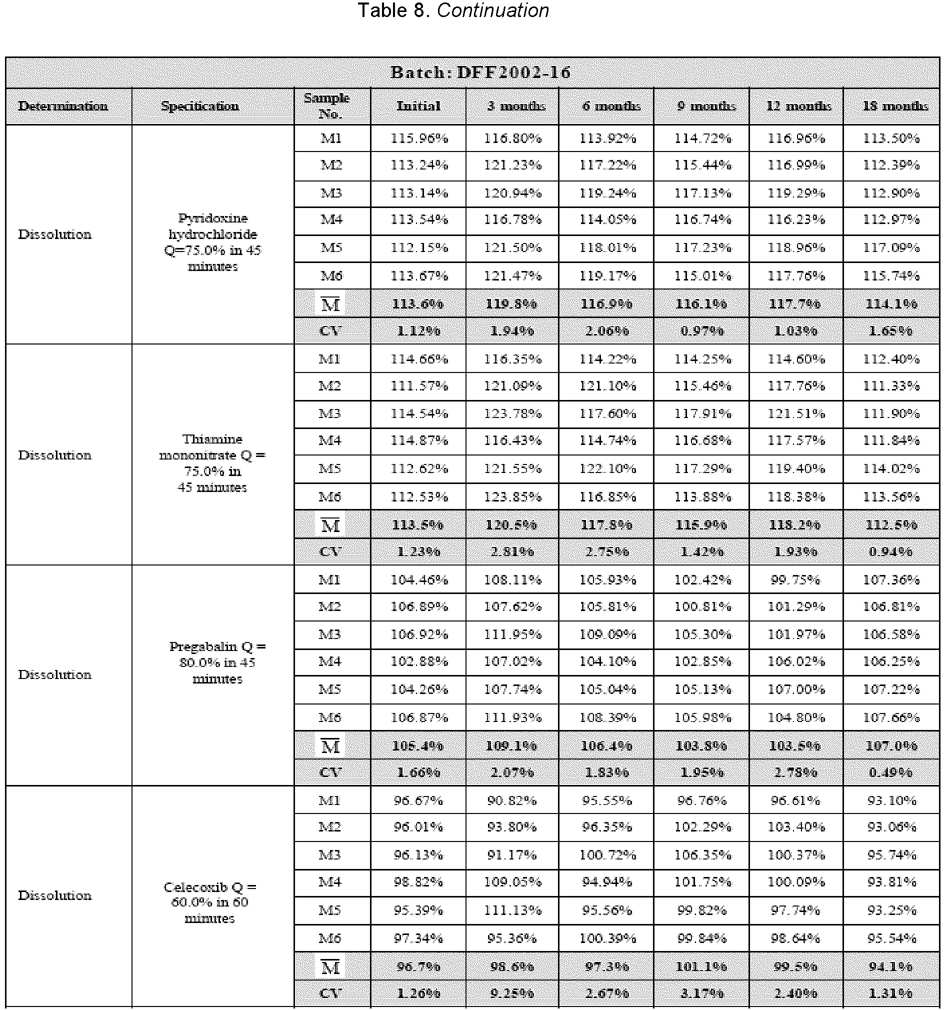

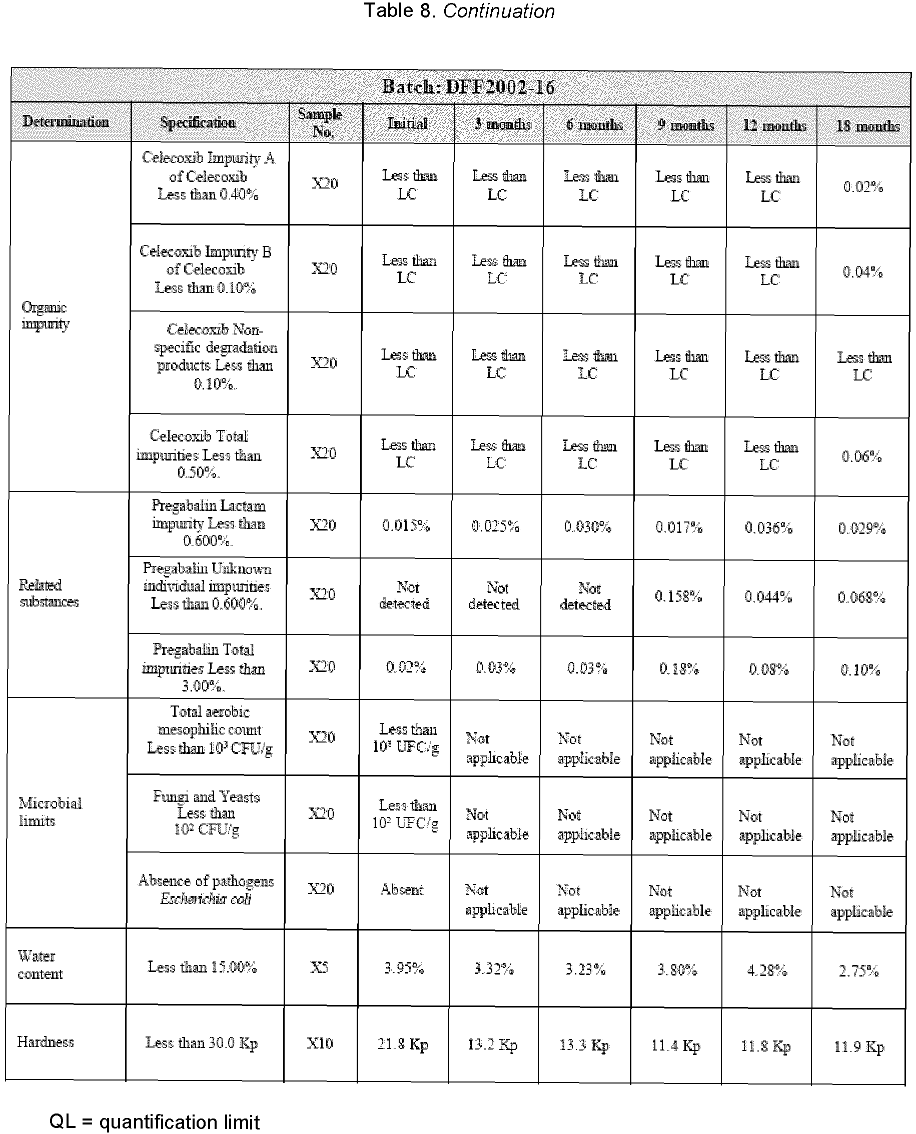

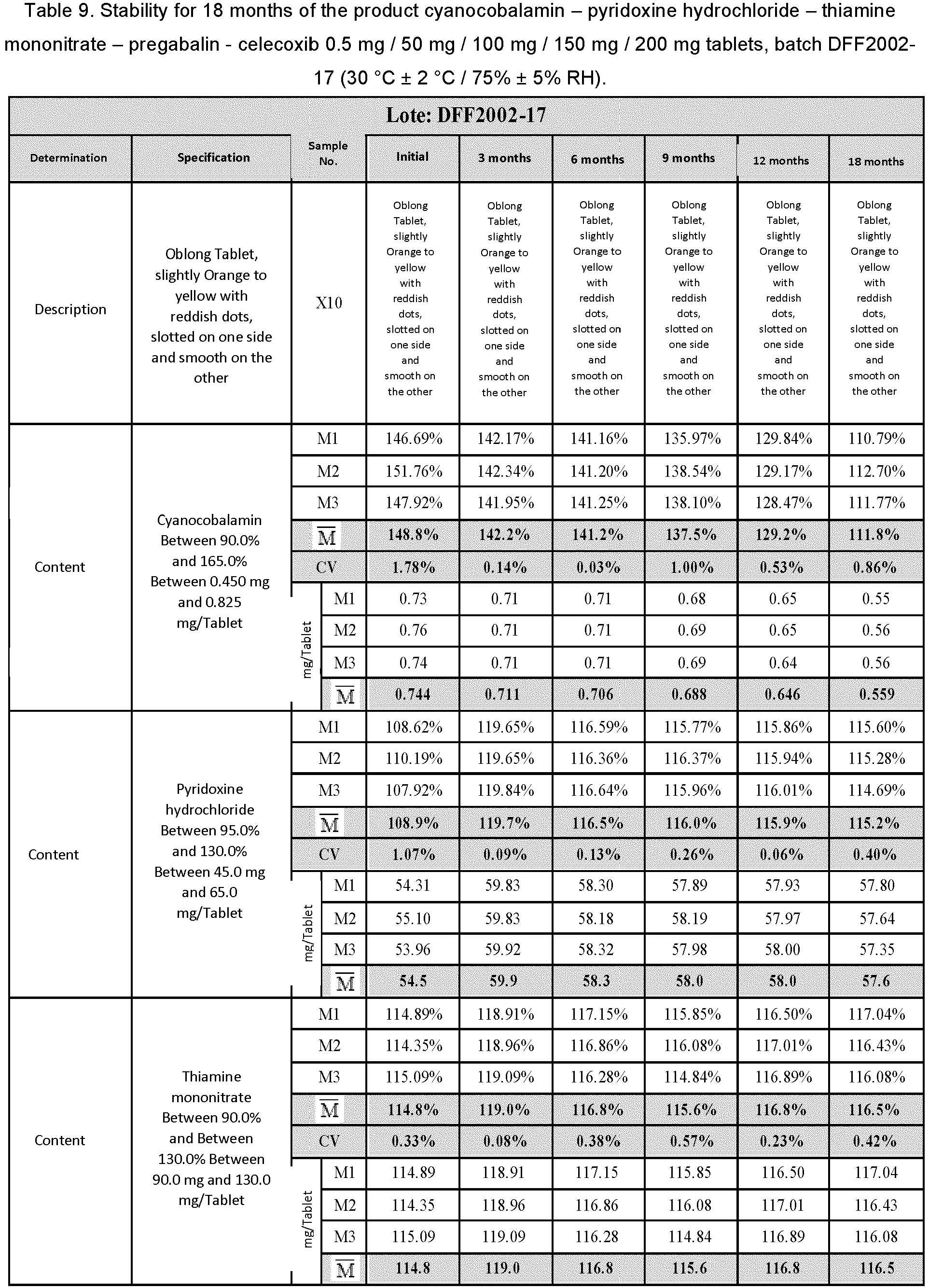

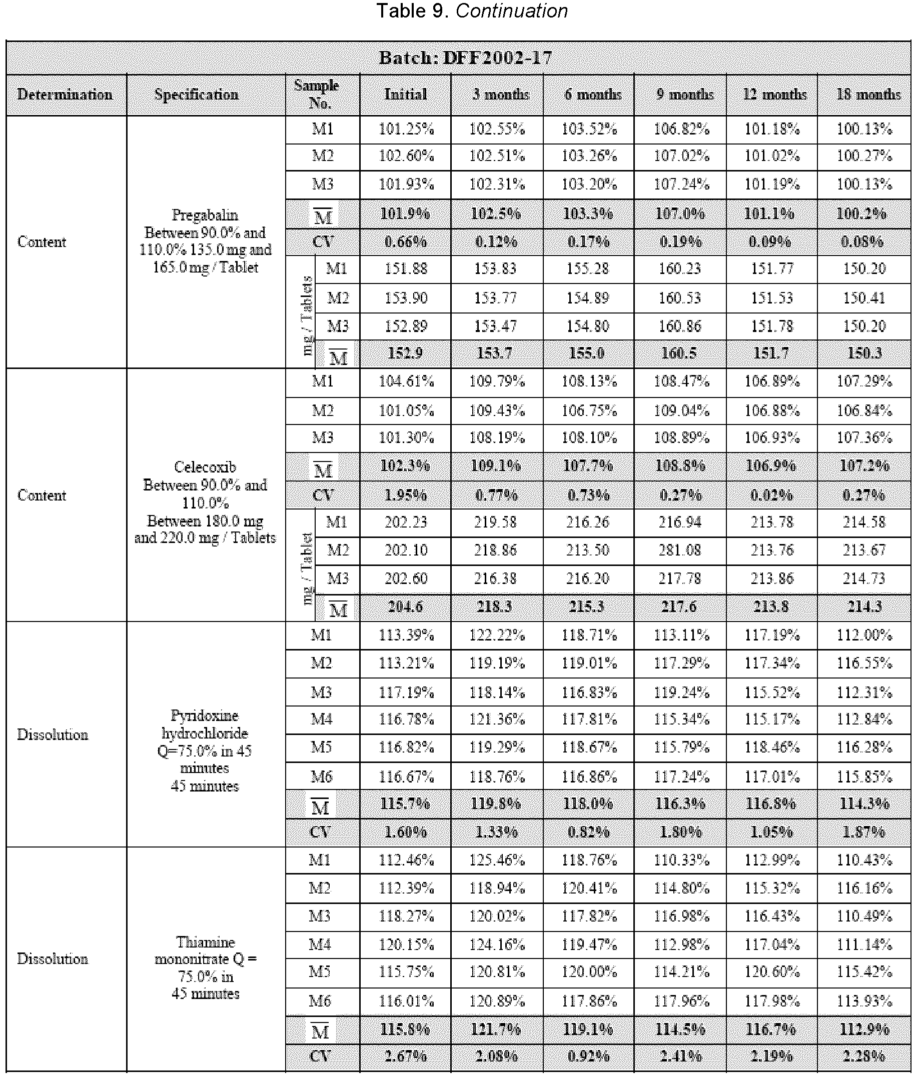

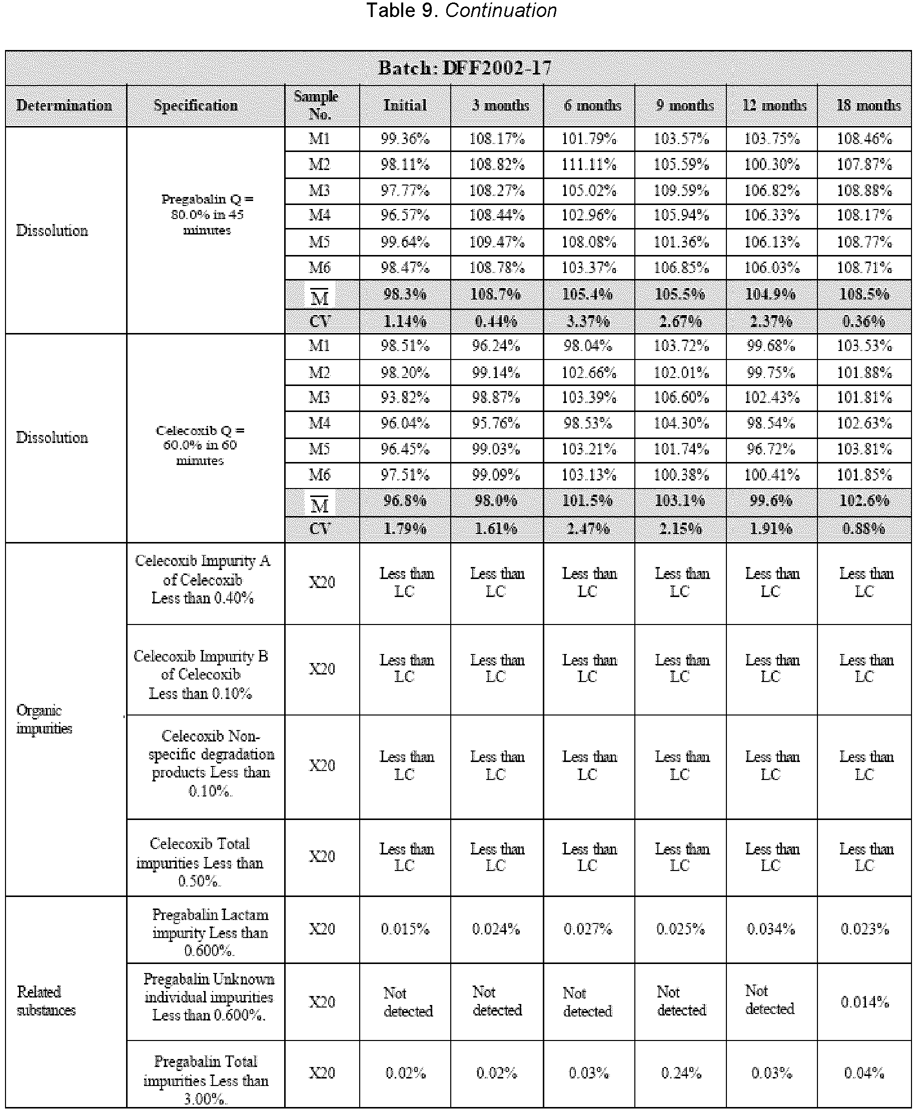

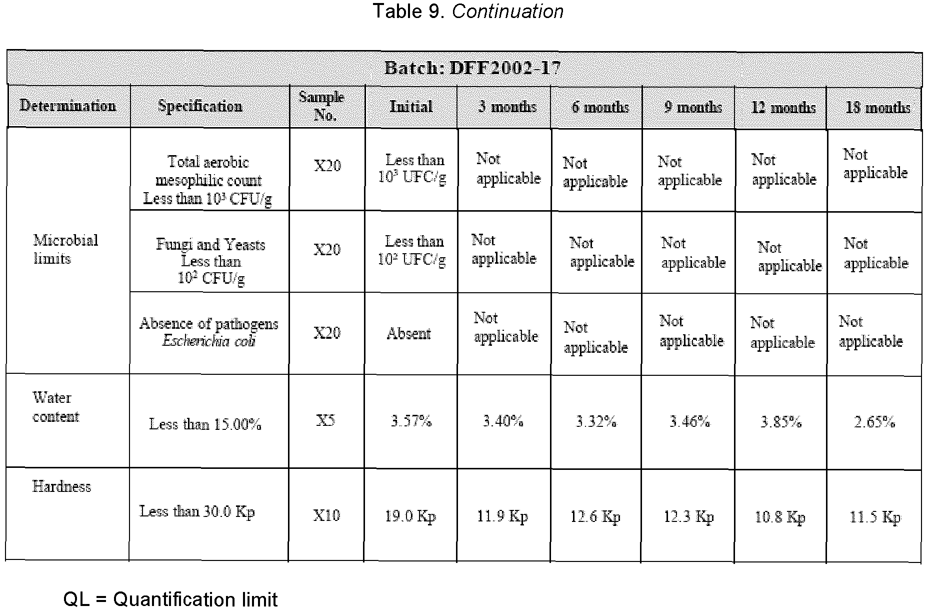

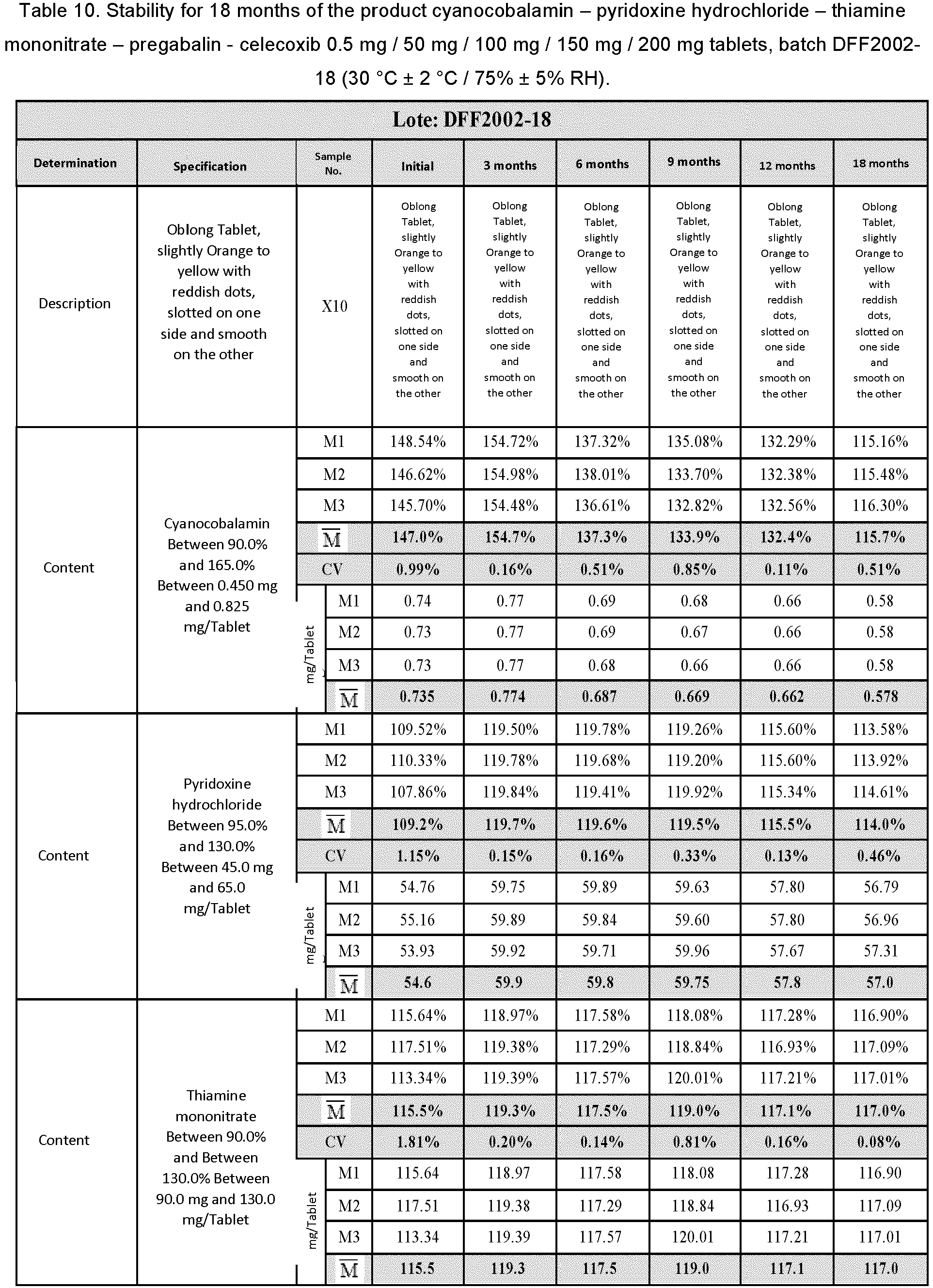

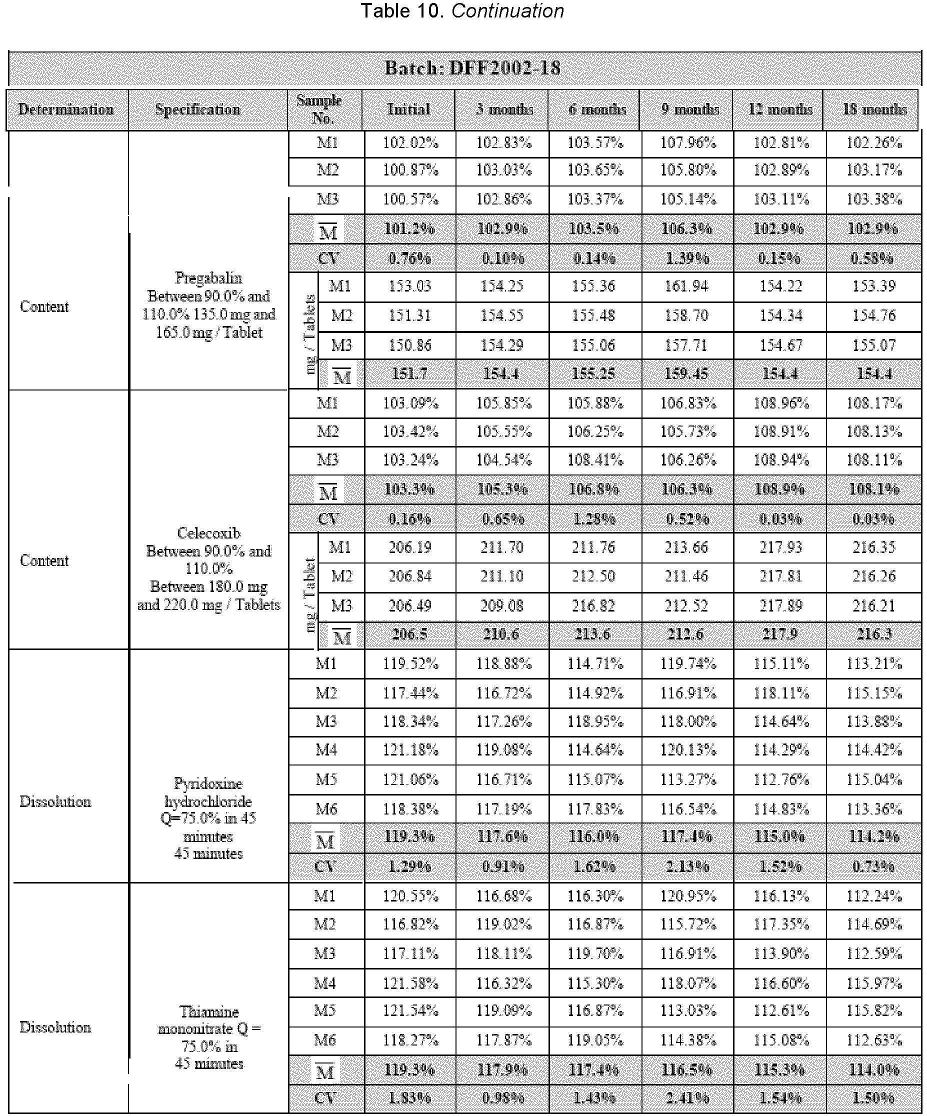

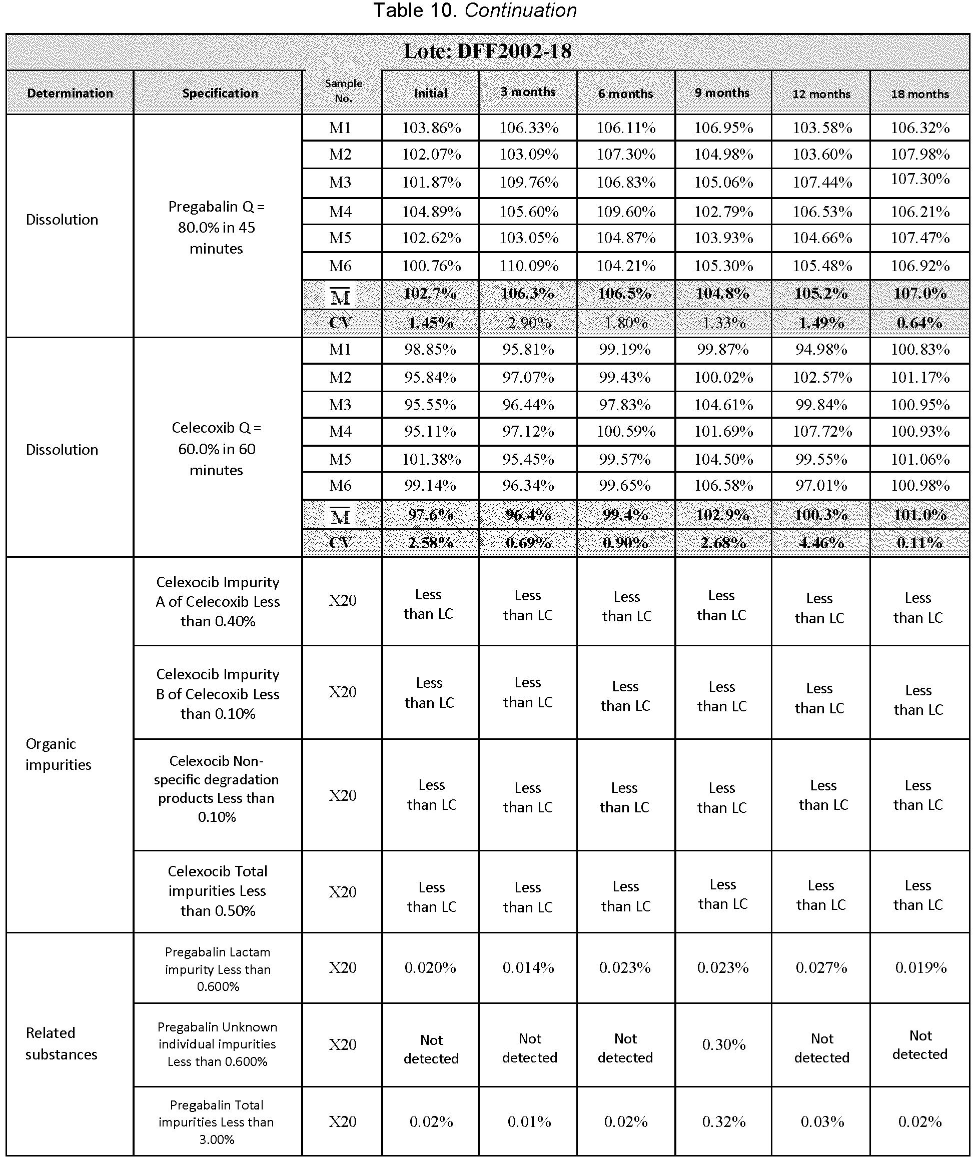

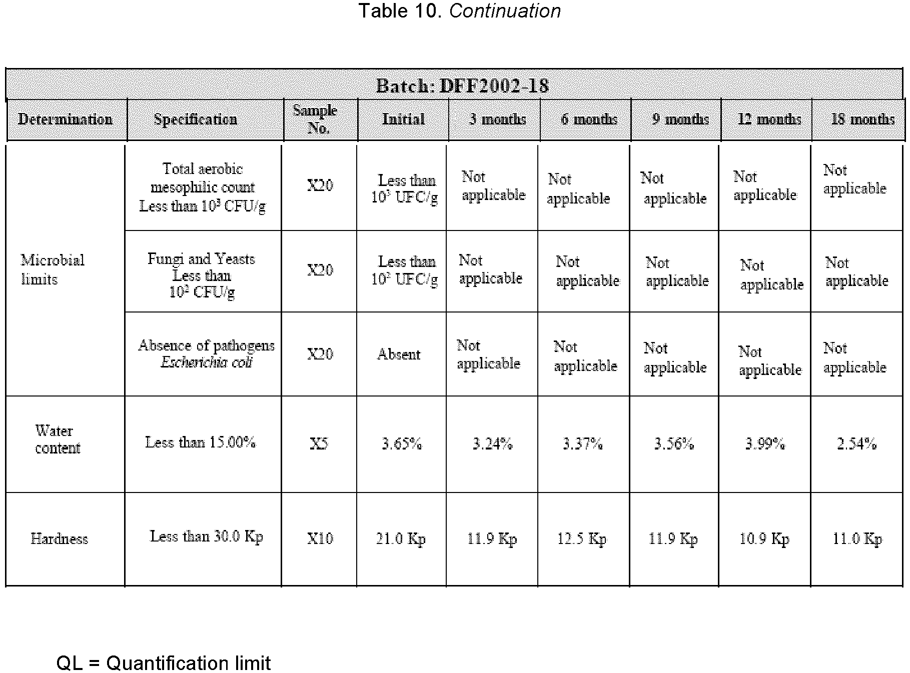

According to the results obtained in the stability study of the product cyanocobalamin - pyridoxine hydrochloride - thiamine mononitrate - pregabalin - celecoxib 0.5 mg / 50 mg / 100 mg / 150 mg / 200 mg tablets, carried out in three pilot batches and packaged in cold-formed aluminum blister 176 µm / aluminum foil 25 µm in a cardboard box, subjected to the long-term stability condition for climatic zone IVB (30 °C ± 2 °C / 75% ± 5% RH) for 18 months for batches DFF2002-16, DFF2002-17 and DFF2002-18 comply with the stability, preserving their chemical and physical characteristics within the specifications established for this product, thus ensuring its quality, efficacy and safety. Tendencies in the content and dissolution results of the active ingredients are also shown (Figures 5 to 13).

The definition of the manufacturing process in combination with the selection of additives is key to achieving adequate bioavailability of celecoxib, which requires an alkaline microenvironment using sodium bicarbonate to control the pH of the pharmaceutical form so that the stomach environment does not affect its absorption.

The combination in a single stable and immediate-release pharmaceutical form for the manufacture of a medicament comprising the drugs or active substances cyanocobalamin, pyridoxine hydrochloride, thiamine mononitrate, pregabalin and celecoxib and pharmaceutically acceptable vehicles will allow greater analgesia at reduced doses and a decrease in adverse effects in the treatment and control of pain with a neuropathic and nociceptive component, for example, where the neuropathy is acute, painful and recurrent.

### Advantages of the invention and industrial application

The present invention relates to a simple optimized method that allows the combined manufacture of five active substances: cyanocobalamin, pyridoxine hydrochloride, thiamine mononitrate, pregabalin and celecoxib in a single solid, stable, immediate release pharmaceutical form for pain. The combined use of drugs that act at distinct levels is associated with greater pain relief and, as they can be used at lower doses, with a reduced risk of adverse effects. In this context, the combined use of B vitamins plus pregabalin and celecoxib would be associated with greater analgesic activity through complementary action mechanisms.

Thus, the combination in a single stable and immediate-release pharmaceutical form for the manufacture of a medicament that includes the drugs or active substances cyanocobalamin, pyridoxine hydrochloride, thiamine mononitrate, pregabalin and celecoxib would be useful in the treatment and control of various pathologies that present with pain with a neuropathic and nociceptive component.

On the other hand, the method for obtaining the pharmaceutical composition of the present invention allows obtaining a single product that combines the dosage of the drugs cyanocobalamin, pyridoxine hydrochloride, thiamine mononitrate, pregabalin and celecoxib, which represents a set of important technological challenges due to the physicochemical properties of each active substance and the difference in dosage, to guarantee obtaining a stable product. It is important to mention that if one of the unit operations is eliminated or its order is modified in the manufacturing process, a stable product is not obtained, and it would not comply with the established quality characteristics.

## Claims

1. A pharmaceutical composition comprising cyanocobalamin, pyridoxine, thiamine, pregabalin and celecoxib wherein the pharmaceutical composition is a single solid, immediate-release pharmaceutical form.

2. The pharmaceutical composition according to claim 1, wherein the pharmaceutical form is a tablet.

3. The pharmaceutical composition according to claim 1 or 2, comprising a dose of 0.5 mg of cyanocobalamin, 50 mg of pyridoxine, 100 mg of thiamine, 150 mg of pregabalin and 200 mg of celecoxib.

4. The pharmaceutical composition according to any one of claims 1 to 3, further comprising at least one pharmaceutically acceptable vehicle selected from the group consisting of a surfactant and/or humectant, a diluent, a solubilizer, a binder, a disintegrant, a dye, an alkalinizing, a lubricant and a solvent.

5. The pharmaceutical composition according to claim 4, wherein the surfactant and/or humectant is selected from the group consisting of sodium lauryl sulfate, poloxamers, castor oil derivatives, polyoxyethylene, benzalkonium chloride, benzethonium chloride, polyoxyethylene alkyl ethers and polyoxyethylene sorbitan fatty acid esters.

6. The pharmaceutical composition according to claim 4, wherein the binder is selected from the group consisting of povidones in all K grades and their derivatives, cellulose derivatives such as carbomers, hydroxypropyl celluloses, carboxymethyl celluloses, starch derivatives such as pregelatinized starch and corn starch.

7. The pharmaceutical composition according to claim 4, wherein the diluent is selected from the group consisting of cellulose derivatives such as microcrystalline cellulose, phosphate derivatives such as dibasic calcium phosphate, starch derivatives such as pregelatinized starch and corn starch and lactose such as anhydrous lactose.

8. The pharmaceutical composition according to claim 4, wherein the disintegrant is selected from the group consisting of cellulose derivatives such as hydroxypropyl celluloses, carboxymethyl celluloses, microcrystalline cellulose, croscarmellose sodium; povidone derivatives such as crospovidone, plastons; starch derivatives such as pregelatinized starch, sodium starch glycolate and corn starch.

9. The pharmaceutical composition according to claim 4, wherein the lubricant is selected from the group consisting of stearate derivatives such as magnesium stearate, zinc stearate, calcium stearate, stearic acid, monostearates, stearyl fumarate; sulfated derivatives such as magnesium lauryl sulfate and magnesium lauryl sulfate, and talc.

10. The pharmaceutical composition according to claim 4, wherein the dye is selected from the group consisting of beta-carotene; indigo carmine, iron oxides, sunset yellow FCF, yellow No. 5, and titanium dioxide.

11. The pharmaceutical composition according to claim 4, wherein the solubilizer is selected from the group consisting of various grades of polyethylene glycol, glyceryl behenate, microcrystalline wax, stearoyl polyoxyglycerides and poloxamer.

12. The pharmaceutical composition according to claim 4, wherein the alkalinizing is selected from the group consisting of sodium carbonate, calcium hydroxide, potassium bicarbonate, sodium bicarbonate, potassium citrate, potassium hydroxide, sodium citrate dihydrate and sodium hydroxide.

13. The pharmaceutical composition according to any one of claims 4 to 12, wherein the surfactant and/or humectant is sodium lauryl sulfate; the binder is a polyvidone; the diluent is selected from the group comprising lactose and/or microcrystalline cellulose; the disintegrant is croscarmellose sodium; the alkalinizing is a bicarbonate; the lubricant is a stearate; the solubilizer is a poloxamer; the dye is yellow No. 5; and the solvent is water.

14. The pharmaceutical composition according to any of claims 1 to 13, comprising:
- 0.50 mg of cyanocobalamin as active ingredient,
- 50.00 mg of pyridoxine as active ingredient,
- 100.00 mg of thiamine as active ingredient,
- 150.00 mg of pregabalin as active ingredient,
- 200.00 mg of celecoxib as active ingredient,
- 7.80 mg of sodium lauryl sulfate as surfactant/humectant,
- 200.00 mg of lactose monohydrate as first diluent,
- 25.00 mg of poloxamer as solubilizer,
- 40.00 mg of povidone as binder,
- 50.00 mg of croscarmellose sodium as disintegrant,
- 342.90 mg of microcrystalline cellulose as a second diluent,
- 3.80 mg of sodium bicarbonate as an alkalinizing,
- 5.00 mg of magnesium stearate as a lubricant, and
- 0.068 mL of purified water as a solvent.

15. A process for the manufacture of a pharmaceutical composition as that described in any one of claims 1 to 14, comprising the steps of:
i. mixing for 10 minutes the active ingredient celecoxib with a first diluent, a disintegrant, a binder, a solubilizer and an alkalinizing, previously sifted;
ii. moistening with purified water and granulating;
iii. idrying the granule obtained from the previous step until reaching a humidity less than or equal to 0.8%;
iv. adding and mixing the active ingredient cyanocobalamin and a portion of a second diluent;
v. adding and mixing for 5 minutes a portion of a second diluent;
vi. repeating the previous step by adding and mixing for 5 minutes a portion of a second diluent;
vii. adding and mixing for 5 minutes a solubilizer;
viii. adding the active ingredients pyridoxine, thiamine, and pregabalin, and a portion of a second diluent;
ix. adding and mixing for 5 minutes a portion of a second diluent;
x. adding and mixing for 5 minutes a binder, a surfactant/humectant and a disintegrant;
xi. adding and mixing for 3 minutes a dye and a lubricant;
xii. compressing the granulate obtained in the previous step.

16. The process according to claim 15, wherein:
- in step i. 50% of the total amount of the disintegrant, 50% of the total amount of the binder, and 50% of the total amount of the solubilizer are mixed;
- in step iv., the portion of the second diluent is 5% of the total amount of the second diluent;
- in step v., the portion of the second diluent is 10% of the total amount of the second diluent;
- in step vi., the portion of the second diluent is 20% of the total amount of the second diluent;
- in step vii., the remaining 50% of the solubilizer is mixed;
- in step viii., 32.5% of the total amount of the second diluent is mixed;
- in step ix., the portion of the second diluent is 32.5% of the total amount of the second diluent;
- in step x., the remaining 50% of the binder and the remaining 50% of the disintegrant are mixed.

17. The process according to claim 15 or 16, wherein the surfactant/humectant is sodium lauryl sulfate, the first diluent is lactose monohydrate, the solubilizer is poloxamer, the binder is polyvidone, the disintegrant is croscarmellose sodium, the second diluent is microcrystalline cellulose, the dye is yellow No. 5, the alkalinizing is sodium bicarbonate, and the lubricant is magnesium stearate.

18. The process according to any one of claims 15 to 17, wherein the pharmaceutical composition is a tablet.

19. A composition as described in any one of claims 1 to 14, for use in the treatment and control of chronic pain.

20. The composition for use according to claim 19, wherein the pain is an acute and recurrent painful neuropathy.
